# EUROPÄISCHE PATENTANMELDUNG

(11) **EP 1 352 606 A1**
(43) Veröffentlichungstag der Anmeldung: **15.10.2003**
(21) Anmeldenummer: 02007962.0
(22) Anmeldetag: 10.04.2002
(51) Int. Cl.: A61B 5/00

(54) **Verfahren zur Überwachung physiologischer Daten eines Individuums, insbesondere zur Fernüberwachung von Patienten**

(71) Anmelder: Heemann, Ulrike, 45239 Essen (DE)
(72) Erfinder: Heemann, Ulrike, 45239 Essen (DE)
(74) Vertreter: Patentanwälte Rehberg + Hüppe

(57) **Zusammenfassung**

Ein Verfahren zur Überwachung physiologischer Daten eines Individuums, insbesondere zur Fernüberwachung von Patienten, weist die Schritte auf:
- Übermitteln von Basisdaten des Individuums von einem Eingabeterminal (1) über eine erste Sendestrecke (5) an einen Überwachungsserver (4),
- Einrichten einer Überwachungsroutine auf dem Überwachungsserver (4) unter Verwendung der Basisdaten, wobei die Überwachungsroutine einer Individualkennung des Individuums zugeordnet ist und mindestens ein Vergleichen von aktuellen physiologischen Daten des Individuums mit Grenzwerten und ein Erzeugen und Aussenden einer Alarmmeldung (13) beim Überschreiten der Grenzwerte umfasst,
- wiederholtes Übermitteln von aktuellen physiologischen Daten und der Individualkennung des Individuums über mindestens eine andere Sendestrecke (8) als die erste Sendestrecke (5) an den Überwachungsserver (4), wobei die mindestens eine andere Sendestrecke (8) eine Telefonverbindung (18) umfasst.

## Beschreibung

Die Erfindung bezieht sich auf ein Verfahren zur Überwachung physiologischer Daten eines Individuums, insbesondere zur Fernüberwachung von Patienten.

Es gibt verschiedene Konstellationen, in denen eine Überwachung physiologischer Daten eines Individuums von Interesse ist. Hierzu gehört beispielsweise die Fernüberwachung von Patienten mit chronischen Krankheiten. Ob der Zustand solcher Patienten stabil ist, kann häufig durch kontinuierliche Überwachung ausgewählter physiologischer Daten bestimmt werden. Änderungen dieser physiologischen Daten können dabei frühzeitig Hinweise auf ein Verlassen des stabilen Zustands geben, dem durch gezielte Behandlungsmaßnahmen entgegenzuwirken ist. Insbesondere dann, wenn diese Behandlungsmaßnahmen nur selten notwendig sind, ist es wünschenswert, den Patienten ein möglichst freies Leben zu ermöglichen und sie nicht für die Überwachung ihrer physiologischen Daten beispielsweise in einem Krankenhaus festzuhalten. Gleichzeitig muss jedoch sichergestellt werden, dass bei Änderungen der physiologischen Daten, die auf eine gefährliche Zustandsänderung des Patienten hinweisen, unverzüglich geeignete Gegenmaßnahmen ergriffen werden.

Man kann sich zu diesem Zweck vorstellen, dass der jeweilige Patient mit einem Sender versehen wird, der die relevanten physiologischen Daten des Patienten direkt in ein Überwachungszentrum sendet, wo die Daten von Fachpersonal laufend überwacht werden. Dies entspräche dem Einsatz von Überwachungsmonitoren für einzelne Patienten in dem Stationsraum einer Krankenhausstation. Ein solches Vorgehen wäre jedoch technisch und personell sehr aufwendig. Zudem würde es dem jeweiligen Patienten nur einen Bewegungsfreiraum innerhalb der Reichweite des Senders erlauben.

Die Überwachung von physiologischen Daten von Individuen ohne Einschränkung der Bewegungsfreiheit der überwachten Individuen wäre auch für verschiedenste Langzeitstudien an Mensch und Tier von Interesse.

Der Erfindung liegt von daher die Aufgabe zugrunde, ein Verfahren zur Überwachung physiologischer Daten eines Individuums aufzuzeigen, das eine große Bewegungsfreiheit des Individuums zulässt und das mit minimalem technischen und personellen Aufwand realisierbar ist.

Die Aufgabe der Erfindung wird durch ein Verfahren mit den Merkmalen des Patentanspruchs 1 gelöst. Vorteilhafte Ausführungsformen dieses Verfahrens sind in den Unteransprüchen 2 bis 12 angegeben.

Kern des neuen Verfahren ist eine Überwachungsroutine auf einem Überwachungsserver. Diese Überwachungsroutine wird unter Verwendung von Basisdaten des jeweiligen Individuums eingerichtet. Hierzu werden die Basisdaten des Individuums zuvor von einem Eingabeterminal über eine erste Sendestrecke an den Überwachungsserver übermittelt. Typischerweise ist das Eingabeterminal ein Computer eines Arztes, der die Basisdaten des Individuums ermittelt hat und die Überwachung startet. Der Computer des Arztes kann dabei temporär über die erste Sendestrecke oder über die erste Sendestrecke als Standleitung mit dem Überwachungsserver verbunden sein. Dabei können durchaus weitere Datenübermittlungsserver zwischengeschaltet sein, indem beispielsweise das Eingabeterminal über eine Telefonverbindung mit einem Internetzugangsserver kommuniziert, der über das Internet mit dem Überwachungsserver in Verbindung steht. Es versteht sich, dass eine Codierung der Basisdaten zur Verbesserung der Datensicherheit bei ihrer Übermittlung über das Internet vorgenommen werden kann und sollte. Die Überwachungsroutine auf dem Überwachungsserver muss mit einer Individualkennung des Individuums verknüpft werden, da auf dem Überwachungsserver eine Vielzahl von Überwachungsroutinen zur Überwachung einer Vielzahl von Individuen gleichzeitig oder zumindest quasi-gleichzeitig ablaufen wird. Es wird keinesfalls die Regel sein, dass auf einem Überwachungsserver nur die Überwachungsroutine für ein einziges Individuum abläuft. Die Überwachungsroutine selbst umfasst mindestens den Vergleich von aktuellen physiologischen Daten des Individuums mit Grenzwerten und ein Erzeugen und Aussenden einer Alarmmeldung beim Überschreiten der Grenzwerte. Die Grenzwerte können starre Grenzwerte sein, die global für jedes Individuum festgelegt werden. Es kann sich auch um individuelle Grenzwerte für das jeweilige Individuum oder umfließenden, beispielsweise von anderen physiologischen Daten abhängige Grenzwerte handeln. Welcher Typ Grenzwert im Einzelfall zu bevorzugen ist, hängt von dem physiologischen Wert ab, der mit diesem Grenzwert zu vergleichen ist, und von dem jeweiligen Individuum bzw. der Zielsetzung der jeweiligen Überwachung. Das Überschreiten des Grenzwerts schließt ein überschreiten des Grenzwerts in einer Richtung von oben nach unten ein, was genauer als Unterschreiten des Grenzwerts zu bezeichnen ist. Wenn die Überwachungsroutine eingerichtet ist, werden mit Ihrer Hilfe die aktuellen physiologischen Daten des Individuums überwacht. Hierzu werden diese physiologischen Daten wiederholt zusammen mit der Individualkennung des Individuums über mindestens eine andere Sendestrecke als die erste Sendestrecke an den Überwachungsserver übermittelt. Die mindestens eine andere Datenstrecke umfasst bei dem neuen Verfahren eine Telefonverbindung. Mit anderen Worten wird das global verfügbare Telefonnetz für die Übermittlung der aktuellen physiologischen Daten des Individuums genutzt, um dem Individuum die gewünschte Bewegungsfreiheit zur Verfügung zu stellen. Dabei ist das neue Verfahren grundsätzlich nicht auf eine Bestimmte Art von Telefonverbindungen beschränkt.

Es versteht sich, dass die Überwachungsroutine nicht nur auf physiologische Daten mit einer Alarmmeldung zu reagieren hat, wenn die Grenzwerte überschritten werden, sondern dass auch das Ausbleiben von erwarteten physiologischen Daten kritisch ist. So kann die Überwachungsroutine ein Aussenden der Alarmmeldung umfassen, wenn eine erwartete Übermittlung von aktuellen physiologischen Daten mit der zugeordneten Individualkennung ausbleibt. Die erwartete Übermittlung kann die Übermittlung in einem bestimmten zeitlichen Maximalabstand zu der letzten erfolgten Übermittlung von physiologischen Daten sein. Dieser Abstand kann starr sein. Er kann aber auch von den zuletzt übermittelten physiologischen Daten abhängig sein. Wenn beispielsweise die zuletzt übermittelten physiologischen Daten einen normalen, unkritischen Zustand eines Patienten anzeigten, kann länger auf die nächste Übermittlung von aktuellen physiologischen Daten gewartet werden, als bei einem schon bedenklichen Zustand. Es ist natürlich nicht erforderlich, dass die Überwachungsroutine unmittelbar nach Ausbleiben einer Übermittlung erwarteter aktueller physiologischer Daten die Alarmmeldung aussendet. Sie kann in diesem Fall zunächst die erwarteten physiologischen Daten nachfragen. Hierzu können die Individualkennung des Individuums oder seine Basisdaten Angaben über eine Telefonverbindung zu dem Individuum umfassen. Es ist festzuhalten, dass alle hier beschriebenen Maßnahmen, soweit nicht ausdrücklich etwas anderes angeführt ist, automatisch, d.h. ohne den Eingriff eines Arztes oder einer anderen Person erfolgen, so dass das neue Verfahren ohne großen Personalaufwand realisierbar und damit besonders kostengünstig ist.

Um dem jeweiligen Individuum zu signalisieren, dass die Überwachung seiner physiologischen Daten erfolgt, so dass es sich beispielsweise sicher fühlen kann, kann die Überwachungsroutine ein Aussenden einer Eingangsbestätigung für jede Übermittlung von aktuellen physiologischen Daten mit der zugeordneten Individualkennung umfassen. Dabei ist es bevorzugt, wenn die Eingangsbestätigung über die bis dahin aufrechterhaltene andere Sendestrecke übermittelt wird. Es ist aber auch denkbar, hierzu eine separate Verbindung zwischen den Überwachungsserver und dem Individuum aufzubauen.

In dem Fall, dass die aktuell übermittelten physiologischen Daten eine Alarmmeldung durch die Überwachungsroutine auslösen, kann die Überwachungsroutine das Aussenden der Alarmmeldung als Antwort auf die Übermittlung der zugrundeliegenden physiologischen Daten umfassen. Hierbei kann es sich um einen unmittelbare Antwort auf die Übermittlung der zugrundeliegenden physiologischen Daten anstelle einer reinen Eingangsbestätigung für die physiologischen Daten handeln. Auch hier ist es bevorzugt, wenn die Antwort über die aufrechterhaltene andere Sendestrecke übermittelt wird. Das Individuum wird damit sofort über seinen kritischen Zustand informiert, so dass sofort vor Ort Gegenmaßnahmen ergriffen werden können. Es versteht sich, dass die eine Übermittlung der Alarmmeldung an das Individuum voraussetzt, dass das Individuum selbst in der Lage ist, die notwendigen Gegenmaßnahmen zu ergreifen.

Für den Fall, dass die notwendigen Gegenmaßnahmen nicht oder nicht allein von dem jeweiligen Individuum oder einer Hilfskraft in seiner Umgebung ergriffen werden können, kann jeder Schritt des Übermittelns von aktuellen physiologischen Daten und der Individualkennung des Individuums an den Überwachungsserver einen Schritt des Übermittelns einer Ortskennung zum Aufenthaltsort des Individuums umfassen. Damit wird es der Überwachungsroutine ermöglicht, die Alarmmeldung mit der Ortskennung an eine Rettungseinrichtung auszusenden, damit von dort Rettungskräfte an den Aufenthaltsort des Individuums geschickt werden.

Die Form und der Inhalt der von der Überwachungsroutine ausgesandten Alarmanmeldung kann unterschiedlich sein. Ein reiner Hinweis auf einen kritischen Zustand ist in der Regel wenig hilfreich. Vorzugsweise umfasst beispielsweise eine an das Individuum ausgesandte Alarmmeldung mindestens eine der folgenden Anweisungen: Übermitteln weiterer physiologischer Daten, Aufnehmen oder Einstellen bestimmter körperlicher Aktivitäten, Einnehmen einer bestimmten Dosis eines bestimmten Medikaments, Rücksprache mit einem Arzt und Begeben in ein Krankenhaus. Zusätzlich ist es sinnvoll, wenn die Überwachungsroutine eine Ausführungsbestätigung für die jeweilige Anweisung abfragt.

Eine weitere Möglichkeit des Aussendens der Alarmmeldung durch die Überwachungsroutine besteht darin, dass diese an einen Arzt geht, um dort eine Handlungsanweisung für das Individuum oder auch eine Rettungseinrichtung abzufragen, die dann über den Überwachungsserver an das Individuum bzw. die Rettungseinrichtung übermittelt wird. Die vollautomatisierte Überwachung der physiologischen Daten einschließlich der vollautomatisierten Maßnahmeneinleitung kann und sollte dann ausgesetzt werden, wenn Zustände bei dem Überwachten Individuum auftreten, welche nicht eindeutig durch bestimmte Gegenmaßnahmen zu bekämpfen sind. Ein Arzt kann auch bei Einrichten der Überwachungsroutine auf dem Überwachungsserver von vornherein festlegen, dass er zeitnah informiert wird, wenn sein Patient einen bestimmten Zustand erreicht. Der Arzt kann dann über den Überwachungsserver die notwendigen Gegenmaßnahmen auslösen. Je nach Aufenthaltsort seines Patienten kann er diesen auch direkt aufsuchen oder durch einen anderen Arzt aufsuchen lassen. Diese Reaktionen des Arztes auf die Alarmmeldung werden von dem Überwachungsserver ebenfalls überwacht, selbst wenn der Überwachungsserver nicht unmittelbar an diesen Gegenmaßnahmen beteiligt ist. Insbesondere bei Gegenmaßnahmen, die von dem Arzt ausgelöst werden und über den Überwachungsserver laufen, ist es bevorzugt, wenn die Überwachungsroutine eine Plausibilitätsprüfung der Handlungsanweisung durch den Arzt vor ihrer Weitergabe beispielsweise an das Individuum umfasst. Im Falle einer unplausiblen Handlungsanweisung kann die Überwachungsroutine beispielsweise eine Bestätigung derselben bei dem Arzt, der die Handlungsanweisung gegeben hat, oder auch einen anderen Arzt als Kontrolle abfragen.

Die an den Überwachungsserver übermittelten physiologischen Daten können in Zuordnung zu der Individualkennung gespeichert werden, um Langzeitprofile der physiologischen Daten aufzuzeichnen. Aus diesen Langzeitprofilen können auch neue Grenzwerte für die Überwachung des jeweiligen Individuums abgeleitet werden. Insbesondere kann der das jeweilige Individuum betreuende Arzt auch noch eine retrospektive Auswertung der physiologischen Daten vornehmen.

Das neue Verfahren eröffnet die Möglichkeit, eine Vielzahl einzelner Individuen mit vergleichsweise geringem technischen und personellen Aufwand zu überwachen bzw. deren physiologischen Daten zu erfassen. Diese Daten können für verschiedenste Auswertungen, Feldstudien und dgl. von Interesse sein. Bei dieser Verwertung der Daten kommt es auf die Angaben zu der Person des konkreten Einzelindividuums nicht an. Diese Angaben sind auch aus Gründen des Datenschutzes geheim zu halten. Es ist daher bevorzugt, wenn die an den Überwachungsserver übermittelten physiologischen Daten zusammen mit nur einem Teil der Basisdaten des Individuums anonymisiert an einen Erfassungsserver übermittelt werden. Auf den Erfassungsserver kann beispielsweise Zugriff gewährt werden, um statistische Auswertungen vorzunehmen. Hiermit ist aber kein Durchgriff auf den Überwachungsserver gegeben, was gegebenenfalls durch geeignete Schutzmaßnahmen sicherzustellen ist.

Die mindestens eine Telefonverbindung, die Teil der anderen Sendestrecke ist, über welche die aktuellen physiologischen Daten an den Überwachungsserver übermittelt werden, kann über eine Festnetzleitung und/oder eine Mobilfunkstrecke führen. Insbesondere bevorzugt ist es, wenn zur Übermittlung der aktuellen physiologischen Daten ein Mobiltelefon verwendet wird. Mit Hilfe des Mobiltelefons ist eine vom Ort des jeweiligen Individuums weitgehend unabhängige Datenübermittlung der physiologischen Daten an den Überwachungsserver möglich.

Zudem verfügen aktuell erhältliche Mobiltelefone bereits über Schnittstellen, über die beispielsweise mit einem Sensor gewonnene physiologische Daten des Individuums drahtlos an das Mobiltelefon übermittelt werden können, so dass sie dann von dem Mobiltelefon über die Telefonverbindung an einen Datenübermittlungsserver übermittelt werden können. Ein besonders gut nutzbarer Schnittstellentyp von Mobiltelefonen wird als wireless LAN-Schnittstelle bezeichnet und ermöglicht die drahtlose Kommunikation eines entsprechend ausgerüsteten Sensors oder auch eines mit einer ebensolchen Schnittstelle versehenen Rechners des Individuums mit dem Mobiltelefon und über dieses mit dem Datenübermittlungsserver. Von dort kann die Weiterübermittlung an den Überwachungsserver beispielsweise in verschlüsselter Form über das Internet erfolgen. Die Individualkennung des jeweiligen Individuums, von dem die physiologischen Daten stammen, kann dabei automatisch und gegebenenfalls ebenfalls in verschlüsselter Form angehängt werden. Dies kann sowohl durch einen Sensor, der die physiologischen Daten gewinnt, als auch durch das Mobiltelefon geschehen. Der ganze Vorgang der Datenübermittlung an den Überwachungsserver kann automatische auf Seiten des Individuums, beispielsweise durch eine intelligente Steuerung seines Mobiltelefons ausgelöst und abgewickelt werden. Es ist aber auch denkbar, dass der Überwachungsserver von sich aus über das Mobiltelefon des Individuums die Sendestrecke bis zu dem die physiologischen Daten gewinnenden Sensor aufbaut.

Neben einer weitgehend automatisierten Übermittlung physiologischen Daten können diese auch von dem Individuum oder einer das Individuum betreuenden Hilfskraft einzeln über eine Tastatur eingegeben oder in ein Mikrophon eingesprochen werden. Dabei kann es sich um die Tastatur bzw. das Mikrophon eines Mobiltelefons oder eines anderen Telefonapparats oder auch um Einrichtungen eines anderen Eingabeterminals, beispielsweise in Form eines Rechners, handeln. Zur Koordination der manuellen bzw. gesprochenen Eingaben kann von dem Übermittlungsserver aus eine übliche Menüführung für die eingebende Person eingesetzt werden. Für gesprochene Eingaben ist eine solche Menüführung beispielsweise im Zusammenhang mit sogenannter Voice Mail-Aktivierung oder Voice-Triggerung bekannt.

Bei einer konkreten Ausgestaltung des neuen Verfahrens kann die Überwachungsroutine auf dem Überwachungsserver beispielsweise in Folge der übermittelten physiologischen Daten des Individuums ein interaktives tasten- oder Voice-getriggertes Abfrageprogramm starten, welches dem Individuum Hinweise zum weiteren Vorgehen gibt. Diese Abfrageprogramm kann auch von einem Dritten, beispielsweise einem Arzt, gestartet werden. Mit dem Abfrageprogramm können dem Überwachungsserver weitergehende Informationen zur Verfügung gestellt werden, die von der Überwachungsroutine nutzbar sind, um dem Individuum individualisierte Anweisungen zu geben. Wenn von dem Individuum beispielsweise Angaben zu zurückliegenden oder zukünftigen Tätigkeiten abgefragt werden, die seine physiologischen Daten potentiell beeinflussen, können diese Angaben von der Überwachungsroutine bei der Bewertung der bisherigen und zukünftigen physiologischen Daten berücksichtigt werden. Für statistische Auswertungen stellen diese zusätzlichen Angaben besonders wertvolle Informationen dar, da sie eine Trennung von verursachten und zufälligen Wertevariationen bei den physiologischen Daten erlauben.

Es kann auch die Möglichkeit vorgesehen sein, dass das Individuum ein Beratungsprogramm, d.h. ein Expertensystem, auf dem Überwachungsserver aufrufen kann, um aktuelle Fragen zu klären. Wenn zum Beispiel ein Diabetespatient abweichend von seinem sonstigen Rhythmus 3 Stunden Sport treiben möchte, kann er abfragen, wie viele Einheiten Insulin er wann dafür spritzen muss. Das Expertensystem kann dabei auch auf die Beurteilung eines Arztes zurückgreifen. In ähnlicher Weise kann dem Individuum auf dem Überwachungsserver ein Lexikon zur Beantwortung allgemeiner Fragen zur Verfügung stehen.

Insbesondere dann, wenn ein Mobiltelefon in die Übermittlung der aktuellen physiologischen Daten an den Überwachungsserver eingebunden ist, ist es sinnvoll, wenn die Individualkennung des Individuums eine Kennung des Mobiltelefons umfasst. Mit der Kennung des Mobiltelefons ist dabei weniger eine Gerätekennung als vielmehr eine Kennung der zum Betrieb des Mobiltelefons eingesetzten Telefonkarte gemeint.

Die Erfindung wird im folgenden anhand eines Ausführungsbeispiels weiter erläutert und beschrieben, dabei zeigt:

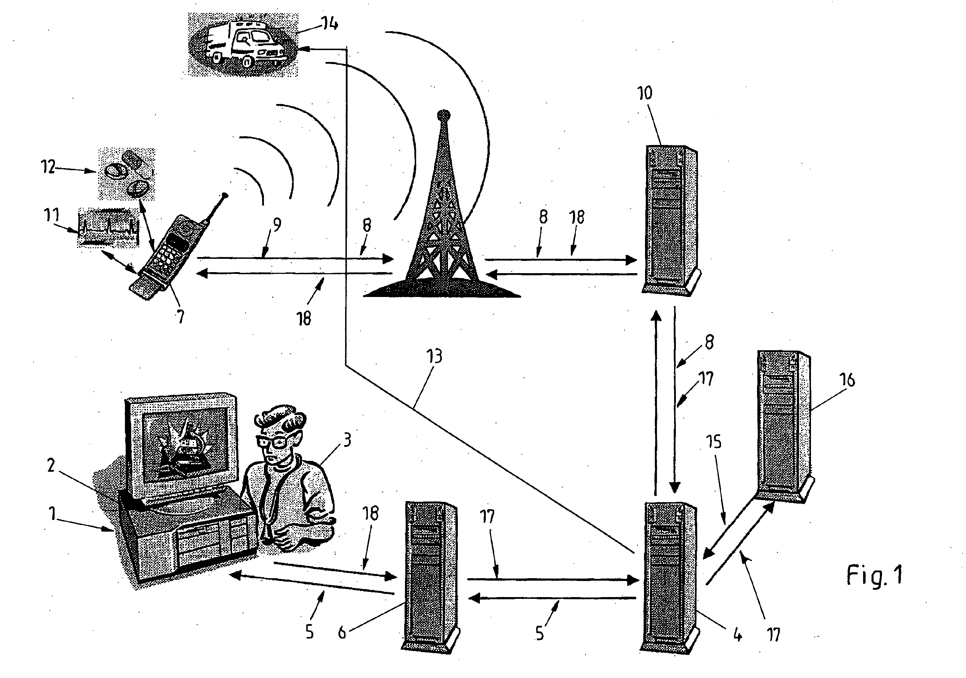

Fig. 1 eine schematische Übersicht über die Datenströme bei der Durchführung des neuen Verfahrens.

In Fig. 1 ist ein Terminal 1 in Form eines Computers 2 dargestellt, mit Hilfe dessen ein Arzt 3 über eine erste Sendestrecke 5 mit einem Überwachungsserver 4 kommuniziert. In die Sendestrecke 5 ist ein Datenübermittlungsserver 6 eingeschaltet. Bis zu dem Datenübermittlungsserver 6, der ein Internetzugangsserver sein kann, kann die Sendestrecke 5 eine Telefonverbindung 18 umfassen und ab dem Datenübermittlungsserver 6 bis zu dem Überwachungsserver 4 eine Internetverbindung 17. Zur Initialisierung der neuen Verfahrens gibt der Arzt 3 über den Terminal 1 Basisdaten eines Patienten ein, die über die Sendestrecke 5 an den Überwachungsserver 4 übermittelt werden. Auf dem Überwachungsserver 4 wird unter Verwendung dieser Basisdaten eine Überwachungsroutine eingerichtet. Die Überwachungsroutine wertet aktuelle physiologische Daten des Patienten aus. Die Zugehörigkeit dieser aktuellen physiologischen Daten zu dem jeweiligen Patienten erfolgt auf Basis einer Individualkennung. Bei der Individualkennung kann es sich um die Rufnummer eines Mobiltelefons 7 handeln, mit dessen Hilfe die Übermittlung der aktuellen physiologischen Daten des Patienten an den Überwachungsserver 4 erfolgt. Diese Übermittlung erfolgt über eine zweite Sendestrecke 8. Teil dieser Sendestrecke ist eine Mobilfunkverbindung 9, die wiederum Teil einer Telefonverbindung 18 ist. Eingeschaltet in die Sendestrecke 8 ist ein weiterer Datenübermittlungsserver 10, der ebenfalls als Internetzugangsserver dienen kann und über eine Internetverbindung 17 in Kontakt mit dem Überwachungsserver steht. Dabei versteht es sich, dass jede Übermittlung von Daten, die durch unbefugte Dritte aus- und verwertbar sind, über das Internet möglichst in codierter, d.h. verschlüsselter Form erfolgen sollte. Bei den über die Sendestrecke 8 übermittelten physiologischen Daten kann es sich beispielsweise um die Pulsfrequenz 11 des Patienten handeln, die von einem Pulsfrequenzsensor, der hier nicht separat dargestellt ist, an eine Schnittstelle des Mobiltelefons 7 und von dort über die Sendestrecke 8 übermittelt wird. Wenn die Pulsfrequenz 11 einen bestimmten Grenzwert von oben oder unten überschreitet, gibt der Überwachungsserver 4 über die Sendestrecke 8 Anweisungen an den Patienten, beispielsweise ein Medikament 12 einzunehmen und diese Einnahme zu bestätigen. Bei einem starken Überschreiten der Grenzwerte kann der Überwachungsserver 4 auch eine Rücksprache mit dem Arzt 3 halten, was unternommen werden soll. Bis auf die unmittelbare Beantwortung einer Rückfrage durch den Arzt erfolgt dies alles voll automatisch. Der Überwachungsserver 4 kann auch eine Alarmmeldung 13 an einen Rettungsdienst 14 aussenden, um dem Patienten sofortige Hilfe von außen zukommen zu lassen. Für die bisher angesprochenen Funktionen ist eine Zuordnung der verfügbaren Daten zu dem jeweiligen Patienten zwingend erforderlich. Der Kreis der Personen, der diese Daten zur Verfügung stehen, ist aber aus Datenschutzgründen möglichst klein zu halten. Umgekehrt besteht aus verschiedenen Gründen ein Interesse an den Daten ohne Ansehen der Person, um beispielsweise statistische Auswertungen vorzunehmen. Hierzu kann der Überwachungsserver 4 über eine weiterer Sendestrecke 15, beispielsweise eine Internetverbindung 17, mit einem Erfassungsserver 16 in Verbindung stehen, auf dem die Daten in anonymisierter Form abgelegt werden. Dabei muss die Schnittstelle des Überwachungsservers 4 zu der Sendestrecke 15 sicherstellen, dass kein Zugriff über den Erfassungsserver 16 auf die personenbezogenen Daten auf dem Überwachungsserver 4 möglich ist.

### BEZUGSZEICHENLISTE

1 - Terminal
2 - Computer
3 - Arzt
4 - Überwachungsserver
5 - Sendestrecke
6 - Datenübermittlungsserver
7 - Mobiltelefon
8 - Sendestrecke
9 - Mobilfunkstrecke
10 - Datenübermittlungsserver

11 - Pulsfrequenz
12 - Medikament
13 - Alarmmeldung
14 - Rettungseinrichtung
15 - Sendestrecke
16 - Erfassungsserver
17 - Internetverbindung
18 - Telefonverbindung

## Patentansprüche

1. Verfahren zur Überwachung physiologischer Daten eines Individuums, insbesondere zur Fernüberwachung von Patienten, mit den Schritten:
- Übermitteln von Basisdaten des Individuums von einem Eingabeterminal (1) über eine erste Sendestrecke (5) an einen Überwachungsserver (4),
- Einrichten einer Überwachungsroutine auf dem Überwachungsserver (4) unter Verwendung der Basisdaten, wobei die Überwachungsroutine einer Individualkennung des Individuums zugeordnet ist und mindestens ein Vergleichen von aktuellen physiologischen Daten des Individuums mit Grenzwerten und ein Erzeugen und Aussenden einer Alarmmeldung (13) beim Überschreiten der Grenzwerte umfasst,
- wiederholtes Übermitteln von aktuellen physiologischen Daten und der Individualkennung des Individuums über mindestens eine andere Sendestrecke (8) als die erste Sendestrecke (5) an den Überwachungsserver (4), wobei die mindestens eine andere Sendestrecke (8) eine Telefonverbindung (18) umfasst.

2. Verfahren nach Anspruch 1, **dadurch gekennzeichnet, dass** die Überwachungsroutine ein Aussenden der Alarmmeldung (13) umfasst, wenn eine erwartete Übermittlung von aktuellen physiologischen Daten mit der zugeordneten Individualkennung ausbleibt.

3. Verfahren nach Anspruch 1 oder 2, **dadurch gekennzeichnet, dass** die Überwachungsroutine ein Aussenden einer Eingangsbestätigung auf jede Übermittlung von aktuellen physiologischen Daten mit der zugeordneten Individualkennung umfasst, wobei die Eingangsbestätigung über die aufrechterhaltene andere Sendestrecke (8) übermittelt wird.

4. Verfahren nach Anspruch 1, 2 oder 3, **dadurch gekennzeichnet, dass** die Überwachungsroutine das Aussenden der Alarmmeldung als Antwort auf die Übermittlung der zugrundeliegenden physiologischen Daten umfasst, wobei die Antwort über die aufrechterhaltene andere Sendestrecke (8) übermittelt wird.

5. Verfahren nach einem der Ansprüche 1 bis 4, **dadurch gekennzeichnet, dass** jeder Schritt des Übermittelns von aktuellen physiologischen Daten und der Individualkennung des Individuums an den Überwachungsserver (4) einen Schritt des Übermittelns einer Ortskennung zum Aufenthaltsort des Individuums umfasst, wobei die Überwachungsroutine die Alarmmeldung (13) mit der Ortskennung an eine Rettungseinrichtung (14) aussendet.

6. Verfahren nach einem der Ansprüche 1 bis 5, **dadurch gekennzeichnet, dass** Überwachungsroutine das Aussenden der Alarmmeldung an das Individuum umfasst, wobei die Alarmmeldung mindestens eine der folgenden Anweisungen:
- Übermitteln weitere physiologischer Daten,
- Aufnehmen oder Einstellen bestimmter körperlicher Aktivitäten,
- Einnehmen einer bestimmten Dosis eines bestimmen Medikaments (12),
- Rücksprache mit einem Arzt (3) und
- Begeben in ein Krankenhaus
umfasst und wobei die Überwachungsroutine eine Ausführungsbestätigung für die Anweisung abfragt.

7. Verfahren nach einem der Ansprüche 1 bis 6, **dadurch gekennzeichnet, dass** die Überwachungsroutine das Aussenden der Alarmmeldung an einen Arzt (3) und ein Abfragen einer Handlungsanweisung für das Individuum von dem Arzt und eine Weitergabe der Handlungsanweisung an das Individuum umfasst.

8. Verfahren nach Anspruch 7, **dadurch gekennzeichnet, dass** die Überwachungsroutine eine Plausibilitätsprüfung der Handlungsanweisung durch den Arzt (3) vor ihrer Weitergabe an das Individuum und ein Abfragen einer Bestätigung einer unplausiblen Handlungsanweisung bei demselben oder einem anderen Arzt umfasst.

9. Verfahren nach einem der Ansprüche 1 bis 8, **dadurch gekennzeichnet, dass** die an den Überwachungsserver (4) übermittelten physiologischen Daten in Zuordnung zu der Individualkennung gespeichert werden.

10. Verfahren nach einem der Ansprüche 1 bis 9, **dadurch gekennzeichnet, dass** die an den Überwachungsserver (4) übermittelten physiologischen Daten zusammen mit einem Teil der Basisdaten des Individuums anonymisiert an einen Erfassungsserver (16) übermittelt werden.

11. Verfahren nach einem der Ansprüche 1 bis 10, **dadurch gekennzeichnet, dass** die mindestens eine Telefonverbindung (18), die Teil der anderen Sendestrecke ist, über eine Festnetzleitung und/oder eine Mobilfunkstrecke (9) führt.

12. Verfahren nach Anspruch 11, **dadurch gekennzeichnet, dass** zumindest ein Teil der physiologischen Daten des Individuums drahtlos an eine Schnittstelle eines Mobiltelefons (7) und von dem Mobiltelefon (7) über die Telefonverbindung (18) an einen Datenübermittlungsserver (10) übermittelt wird.
